# EUROPEAN PATENT APPLICATION

(11) **EP 4 454 681 A1**
(43) Date of publication of application: **30.10.2024**
(21) Application number: 22911533.2
(22) Date of filing: 30.09.2022
(51) Int. Cl.: A61M 5/145, A61M 5/142

(54) **ELECTROOSMOTIC PUMP**

(30) Priority: 24.12.2021 KR 20210187465
(71) Applicant: Eoflow Co., Ltd., Seongnam-si, Gyeonggi-do 13605 (KR)
(72) Inventor: LEE, Do Kyung, Hwaseong-si Gyeonggi-do 18466 (KR); JEONG, Jun Hyuk, Yongin-si Gyeonggi-do 16918 (KR)
(74) Representative: Pfenning, Meinig & Partner mbB
(86) International application number: PCT/KR2022/014732
(87) International publication number: WO 2023/120894

(57) **Abstract**

An embodiment of the present disclosure may provide an electroosmotic pump including: a housing having a shaft hole; a shaft passing through the shaft hole, arranged to extend inside and outside the housing, and capable of a reciprocating motion in an extension direction thereof; and a moving member coupled to the shaft outside the housing, wherein the shaft may be press-fittable into the moving member.

## Description

### Technical Field

The present disclosure relates to an electroosmotic pump, and more particularly, to an electroosmotic pump using a fluid.

### Background Art

Generally, liquid medicine injection devices, such as insulin injection devices, are used to inject liquid medicine into the body of a patient. Such liquid medicine injection devices are used by professional medical staffs, such as medical doctors and nurses. However, in most cases, liquid medicine injection devices are used by the general public, such as patients themselves or guardians thereof.

In this regard, patch-type liquid medicine injection devices that are easily used while being attached to the human body for a certain period of time are under development. Such liquid medicine injection devices may be used while being attached to the body of the patient, such as the abdomen or waist of the patient, in the form of a patch for a certain period of time.

A liquid medicine injection device may include a driving member, such as an electroosmotic pump, so as to actively inject liquid medicine. The electroosmotic pump is a pump that utilizes a fluid movement phenomenon occurring when a voltage is applied across a capillary tube or a porous separation film.

The electroosmotic pump includes a shaft that is capable of performing a linear reciprocating motion according to the movement of fluid therein, and a moving member that is coupled to the shaft to perform a linear reciprocating motion along with the shaft and transmit power to a liquid medicine discharge mechanism.

### Disclosure

### Technical Problem

Various types of driving members, such as motors or pumps, may be used in a mechanism for driving a medicine injection device such as an insulin injection device. The present disclosure relates to a driving member capable of fine pumping by using a fluid and is intended to shorten a device manufacturing time and increase yield by improving processes related to a shaft and a moving member. However, this is only an example and the scope of the present disclosure is not limited thereby.

### Technical Solution

As a means for solving the technical problems described above, an electroosmotic pump according to an embodiment of the present disclosure may include: a housing having a shaft hole; a shaft passing through the shaft hole, arranged to extend inside and outside the housing, and capable of a reciprocating motion in an extension direction thereof; and a moving member coupled to the shaft outside the housing, wherein the shaft may be press-fittable into the moving member.

In an embodiment, the shaft may be press-fitted into a through-hole formed in the moving member in the extension direction of the shaft, a first fastening part, a second fastening part, a third fastening part may be sequentially formed in the extension direction of the shaft at one end portion of the shaft coupled to the moving member, the first fastening part may be contactable with one surface of the moving member, the third fastening part may be contactable with another surface opposite to the one surface of the moving member, and the second fastening part may be located in the through-hole and connects the first fastening part to the third fastening part.

In an embodiment, the through-hole may include: a first through-hole part having a first diameter on one side of the moving member; and a second through-hole part having a second diameter relatively greater than the first diameter on another side of the moving member, a diameter of the first fastening part may correspond to the second diameter, and a diameter of the third fastening part may be relatively greater than the second diameter.

In an embodiment, a portion of the second fastening part may correspond to the first diameter, and a remaining portion of the second fastening part may correspond to the second diameter.

In an embodiment, the shaft may be press-fitted from a side into an insertion groove formed in the moving member, a first fastening part, a second fastening part, and a third fastening part may be sequentially formed in the extension direction of the shaft at one end portion of the shaft coupled to the moving member, the first fastening part may be contactable with one surface of the moving member, the third fastening part may be contactable with another surface opposite to the one surface of the moving member, and the second fastening part may be located in the insertion groove and connects the first fastening part to the third fastening part.

In an embodiment, the insertion groove may include: an insertion part to which the second fastening part is inserted and fixed; a guide part expanded and opened in an outward direction from the insertion part; and an engagement part protruding inward between the insertion part and the guide part.

In an embodiment, a diameter of each of the first fastening part and the third fastening part may be relatively greater than a diameter of the insertion part.

In an embodiment, the insertion groove may further include an engagement alleviation part that is opened from the insertion part to an outside of the moving member, and a minimum gap formed by the engagement alleviation part may be equal to a diameter of the insertion part.

Aspects, features, and advantages other than those described above will become better understood through the accompanying drawings, the claims, and the detailed description.

### Advantageous Effects

According to an electroosmotic pump according to an embodiment of the present disclosure, a working time may be shortened by improving a manufacturing process, and thus, an increase in yield may be expected .

In addition, cost reduction may be expected by reducing the number of parts.

However, these effects are only examples and the scope of the present disclosure is not limited by such effects.

### Description of Drawings

FIG. 1 is a perspective view illustrating an electroosmotic pump according to an embodiment of the present disclosure.
FIG. 2 is a cross-sectional view taken along line X-X' of FIG. 1.
FIGS. 3A to 3C are diagrams of a portion A of FIG. 2.
FIGS. 4A to 4C are diagrams of another embodiment of FIG. 3.
FIGS. 5A and 5B are schematic diagrams illustrating the reaction in a first electrode body and a second electrode body with respect to a membrane, according to an embodiment of the present disclosure.
FIGS. 6A to 6C are cross-sectional views describing a reciprocating motion of a shaft according to an embodiment of the present disclosure.
FIG. 7 is a graph showing a ratio of a volume of a second sub-space to a volume of a first space according to a reciprocating motion of a shaft.

### Mode for Disclosure

As the present description allows for various changes and numerous embodiments, certain embodiments will be illustrated in the drawings and described in detail in the written description. Effects and features of the present disclosure, and methods of achieving them will be clarified with reference to embodiments described below in detail with reference to the drawings. However, the present disclosure is not limited to the following embodiments and may be embodied in various forms.

Hereinafter, embodiments will be described in detail with reference to the accompanying drawings. When describing embodiments with reference to the accompanying drawings, the same or corresponding elements are denoted by the same reference numerals, and redundant descriptions thereof are omitted.

In the following embodiments, the terms "first," "second," etc. are not used in a restrictive sense and are used to distinguish one element from another.

In the following embodiments, the singular forms are intended to include the plural forms as well unless the context clearly indicates otherwise.

In the following embodiments, the terms "comprise" or "include" specify the presence of stated features or elements, but do not preclude the presence or addition of one or more other features or elements.

Sizes of elements in the drawings may be exaggerated or reduced for convenience of explanation. For example, since sizes and thicknesses of elements in the drawings are arbitrarily illustrated for convenience of explanation, the following embodiments are not limited thereto.

In the following embodiments, regions or elements are referred to as being connected to each other, the regions or the elements may be directly connected to each other or indirectly connected to each other with intervening regions or elements therebetween.

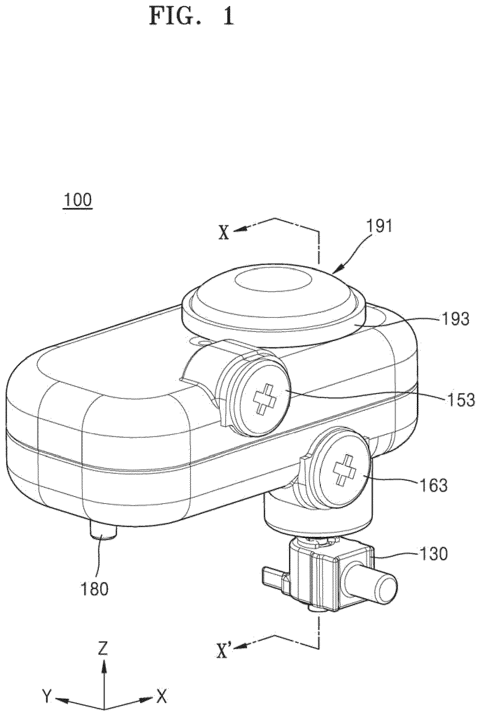

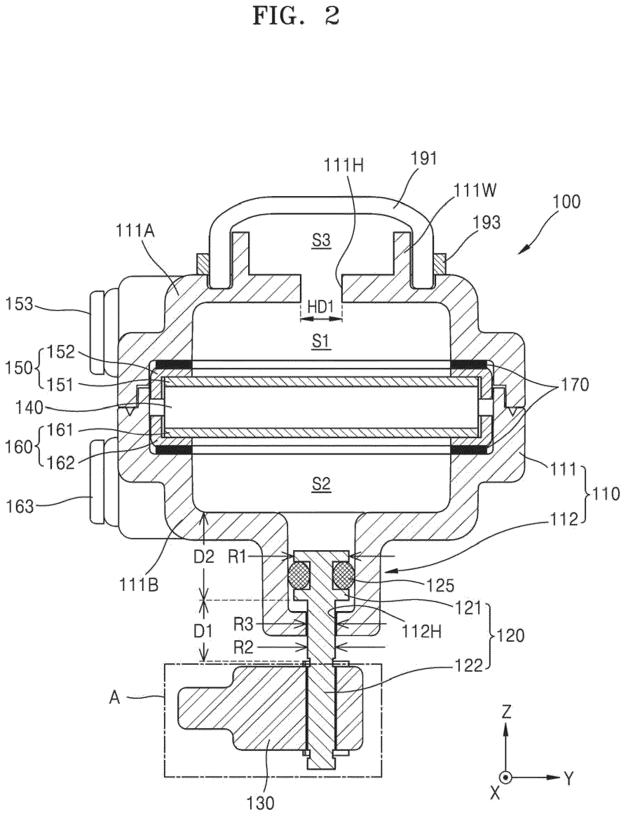

FIG. 1 is a perspective view of an electroosmotic pump according to an embodiment of the present disclosure, and FIG. 2 is a cross-sectional view taken along line X-X' of FIG. 1.

Referring to FIGS. 1 and 2, a housing 110 of an electroosmotic pump (hereinafter referred to as a 'pump') 100 may include a shaft hole 112H provided on one side thereof, and a shaft 120 having a certain length may extend to the outside of the housing 110 through the shaft hole 112H.

As an embodiment, the shaft hole 112H may be formed in a protrusion part 112 extending to one side of a main body 111 of the housing 110, and a diameter of the protrusion part 112 may be formed to be less than a diameter of the main body 111.

A first part 121 of the shaft 120 is arranged inside the housing 110, and a second part 122 passes through the shaft hole 112H and extends to the outside of the housing 110, as described above. The shaft 120 may reciprocate in a vertical direction (a Z direction) in FIGS. 1 and 2.

When the shaft 120 reciprocates, the first part 121 may perform a linear reciprocating motion in the inner space of the housing 110, for example, in the inner space corresponding to the protrusion part 112. Since a diameter R1 of the first part 121 of the shaft 120 is greater than a diameter R3 of the shaft hole 112H, the first part 121 is not separated from the housing 110.

A sealing material 125 may be arranged on the side surface of the first part 121 of the shaft 120. The inner space of the housing 110, for example, the space defined by the inner surface of the housing 110 and the inner surface of the shaft 120, is a sealed space. A fluid exists in the inner space. The sealing material 125 may prevent the fluid from leaking through a gap between the housing 110 and the shaft 120. In FIG. 2, the fluid is omitted for convenience of explanation.

According to an embodiment, as illustrated in FIG. 2, the sealing material 125 may cover the side surface of the first part 121 in the form of an O-ring, and the sealing material 125 may prevent the fluid existing inside the housing 110 from leaking to the outside of the housing 110 through the shaft hole 112H.

The leakage of the fluid may be prevented more effectively by making a first distance D1 from the first part 121 of the shaft 120 to a moving member 130 equal to or less than an inner length D2 of the protrusion part 112.

The second part 122 of the shaft 120 may have a diameter R2 less than the diameter R3 of the shaft hole 112H. One end of the second part 122 may be coupled to the moving member 130 in the outside of the housing 110. The moving member 130 may reciprocate in the vertical direction along with the motion of the shaft 120. The motion of the moving member 130 may be converted into power for injecting liquid medicine into a liquid medicine discharge mechanism of a liquid medicine injection device on which a pump is mounted.

A membrane 140 may be arranged in the inner space of the housing 110, for example, in the inner space corresponding to the main body 111. The inner space includes a first space S1 and a second space S2 respectively located on both sides of the membrane 140.

In FIG. 2, a space far from the shaft 120 with respect to the membrane 140 is represented as the first space S1, and a space adjacent to the shaft 120 with respect to the membrane 140 is represented as the second space S2.

The membrane 140 may have a porous structure that allows the fluid and ions to move. The membrane 141 may be, for example, a frit-type membrane manufactured by sintering spherical silica with heat. For example, the spherical silica used to form the membrane may have a diameter of about 20 nm to about 500 nm, specifically about 30 nm to about 300 nm, and more specifically, about 40 nm to about 200 nm.

When the diameter of the spherical silica satisfies the range described above, pressure due to the first fluid passing through the membrane 140, that is, pressure sufficient to move the shaft 120, may be generated.

In the embodiment described above, it has been described that the membrane 140 includes the spherical silica, but the membrane 140 is not limited thereto.

In another embodiment, the type of the membrane 140 is not limited as long as the membrane 140 is formed of a material capable of causing an electrokinetic phenomenon due to zetapotential, such as porous silica or porous alumina.

The membrane 140 may have a thickness of about 20 µm to about 10 mm, specifically about 300 µm to about 5 mm, and more specifically, about 1,000 µm to about 4 mm.

A first electrode body 150 and a second electrode body 160 may be respectively arranged on both sides of the membrane 140. The first electrode body 150 may include a first porous plate 151 and a first strip 152 arranged on a first side of the membrane 140. The second electrode body 160 may include a second porous plate 161 and a second strip 162 arranged on a second side of the membrane 140.

The first porous plate 151 and the second porous plate 161 may be respectively arranged to be in contact with main surfaces on both sides of the membrane 140. The first porous plate 151 and the second porous plate 161 may effectively move the fluid and ions through the porous structures thereof.

The first porous plate 151 and the second porous plate 161 may have a structure in which an electrochemical reactant is formed in a porous base layer. For example, the electrochemical reactant may be formed by electrodeposition or coating on the porous base layer through methods such as electroless plating, vacuum deposition, coating, and sol-gel process.

The porous base layer may be an insulator. For example, the porous base layer may include one or more selected from non-conductive ceramic, non-conductive polymer resin, non-conductive glass, and any combination thereof.

The non-conductive ceramic may include, for example, one or more selected from the group consisting of rock wool, gypsum, ceramic, cement, and any combination thereof, and specifically, may include one or more selected from the group consisting of rock wool, gypsum, and any combination thereof, but the present disclosure is not limited thereto.

The non-conductive polymer resin may include, for example, one or more selected from the group consisting of: synthetic fiber selected from the group consisting of polypropylene, polyethylene terephthalate, polyacrylonitrile, and any combination thereof; natural fiber selected from the group consisting of wool, cotton, and any combination thereof; sponge; a porous material derived from living organisms, for example, the bones of living organisms; and any combination thereof, but the present disclosure is not limited thereto.

The non-conductive glass may include one or more selected from the group consisting of glass wool, glass frit, porous glass, and any combination thereof, but the present disclosure is not limited thereto.

The porous base layer may have a pore size of about 0.1 µm to about 500 µm, specifically about 5 µm to about 300 µm, and more specifically, about 10 µm to about 200 µm.

When the pore size of the porous support satisfies the range described above, the fluid and ions may be effectively moved, thereby improving the stability, lifespan characteristics, and efficiency of the pump 100.

The electrochemical reactant may include a material that may form a pair of reactions in which an oxidizing electrode and a reducing electrode exchange cations, for example, hydrogen ions during an electrode reaction of the first electrode body 150 and the second electrode body 160, and at the same time, may form a reversible electrochemical reaction.

The electrochemical reactant may include, for example, silver/silver oxide, silver/silver chloride, MnO(OH), polyaniline, polypyrrole, polythiophene, polythionine, quinone-based polymer, and any combination thereof.

The first strip 152 and the second strip 162 may be respectively arranged at the edges of the first porous plate 151 and the second porous plate 161, and may be connected to a first terminal 153 and a second terminal 163 arranged outside the housing 110. The first strip 152 and the second strip 162 may each include a conductive material, such as silver or copper.

The fluid provided in the inner space of the housing 110 may include a first fluid and a second fluid having different phases. The first fluid may include a liquid such as water and the second fluid may include a gas such as air.

The first fluid existing in the inner space does not completely fill the inner space. That is, the volume of the inner space is greater than the volume of the first fluid existing in the inner space. The second fluid may exist in a portion of the inner space where water does not exist.

A sealing material 170 may be arranged on either side of the structure of the membrane 140, the first electrode body 150, and the second electrode body 160. The sealing material 170 may be formed in a ring shape with the area corresponding to the edge of the structure described above.

The fluid described above, for example, the first fluid moves from the first space S1 to the second space S2 or vice versa in the thickness direction of the membrane 140 so as to pass through the membrane 140. At this time, the sealing material 170 may block a gap between the inner surface of the housing 110 and the above-described structure, thereby preventing the fluid from moving into the gap.

The fluid may flow into the inner space through the injection port 180, as illustrated in FIG. 1. As an embodiment, after the entire inner space is filled with the first fluid through the injection port 180 on one side, a part of the first fluid is taken out to the outside through the injection port 180, and then, the injection port 180 is closed. Accordingly, the first fluid and the second fluid may exist in the inner space of the housing 110.

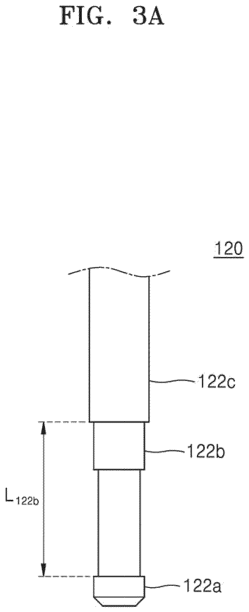

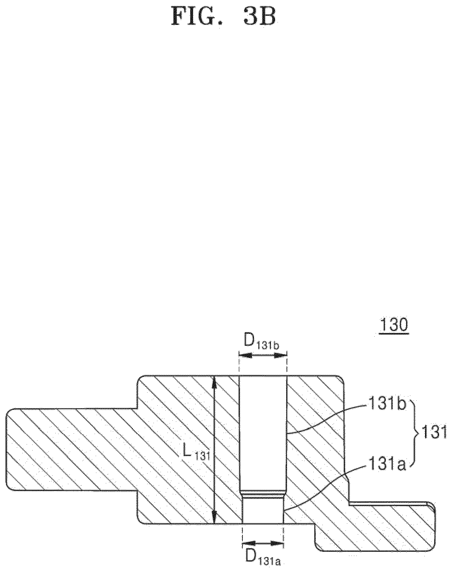

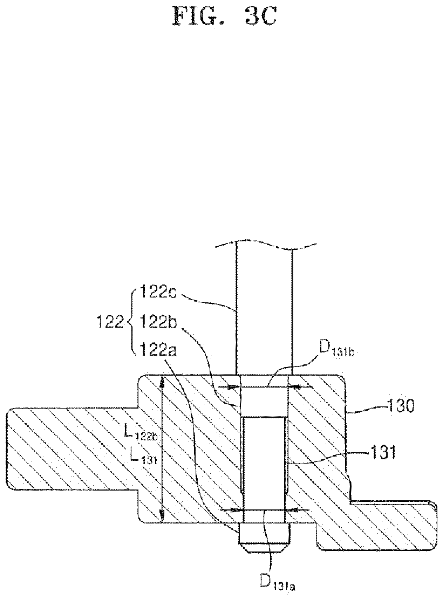

FIG. 3 is a diagram of a portion A of FIG. 2. FIG. 3A is a diagram illustrating a portion of the second part 122 of the shaft 120, FIG. 3B is a diagram illustrating the moving member 130, and FIG. 3C is a diagram illustrating a state in which the shaft 120 and the moving member 130 are coupled to each other.

The second part 122 of the shaft 120 may be coupled to the moving member 130 in the outside of the housing. The shaft 120 and the moving member 130 may be coupled to each other by press-fitting.

Referring to FIG. 3A, the shaft 120 may include a first fastening part 122a, a second fastening part 122b, and a third fastening part 122c. The first fastening part 122a, the second fastening part 122b, and the third fastening part 122c may be sequentially arranged in the extension direction of the shaft 120.

The first fastening part 122a, the second fastening part 122b, and the third fastening part 122c of the shaft 120 may have different diameters. For example, the first fastening part 122a may have a diameter greater than a diameter of the second fastening part 122b and less than a diameter of the third fastening part 122c.

The first fastening part 122a may be formed such that an end portion opposite to the second fastening part 122b is tapered and a diameter of a cross-section thereof gradually decreases toward the end.

The second fastening part 122b may have a certain length L_{122b}. The second fastening part 122b may be formed such that a portion of the second fastening part 122b and a remaining portion excluding the portion have different diameters. That is, the second fastening part 122b may include at least two portions having different diameters.

Referring to FIG. 3B, the moving member 130 may have a through-hole 131 formed to pass through the moving member 130 from one side to the other side. The through-hole 131 may have a certain length L₁₃₁.

The through-hole 131 may include a first through-hole part 131a and a second through-hole part 131b. The first through-hole part 131a may be formed to have a first diameter D₁₃₁ₐ on one side of the moving member 130, and the second through-hole part 131b may be formed to have a second diameter D_{131b} on the other side of the moving member 130. A length of the first through-hole part 131a may be formed to be shorter than a length of the second through-hole part 131b. The first diameter D₁₃₁ₐ may be formed to be less than the second diameter D_{131b}.

A boundary between the first through-hole part 131a and the second through-hole part 131b is tapered, and a diameter from the second through-hole part 131b to the first through-hole part 131a may be formed to gradually decrease from the second diameter D_{131b} to the first diameter D₁₃₁ₐ.

Referring to FIG. 3C, the shaft 120 may be coupled to the moving member 130 by being press-fitted into the through-hole 131 formed in the moving member 130 in a direction from the second through-hole part 131b to the first through-hole part 131a. For example, the diameter of the first fastening part 122a of the shaft 120 corresponds to the second diameter D_{131b}, such that the first fastening part 122a may be slidably inserted into the second through-hole part 131b. The first diameter D₁₃₁ₐ is formed to be less than the second diameter D_{131b}, such that the first fastening part 122a may pass through the first through-hole part 131a due to a force applied in the extension direction at the boundary between the second through-hole part 131b and the first through-hole part 131a. At this time, in order to facilitate press-fitting of the first fastening part 122a, at least one of the end portion of the first fastening part 122a and the boundary between the second through-hole part 131b and the first through-hole part 131a may be tapered. The diameter of the third fastening part 122c is formed to be greater than the second diameter D_{131b} such that, when the first fastening part 122a passes through the first through-hole part 131a, the third fastening part 122c may not be inserted into the second through-hole part 131b.

In a state in which the shaft 120 and the moving member 130 are coupled to each other, the second fastening part 122b of the shaft 120 may be located inside the through-hole 131. The length L_{122b} of the second fastening part 122b corresponds to the length L₁₃₁ of the through-hole 131, the first fastening part 122a may be in contact with one side of the moving member 130, and the third fastening part 122c may be in contact with the other side of the moving member 130. Since the first fastening part 122a and the third fastening part 122c are respectively in contact with one side and the other side of the moving member 130, such that the movement of the moving member 130 may be restricted to prevent the moving member 130 from moving in the extension direction of the shaft 120.

In a state in which the shaft 120 and the moving member 130 are coupled to each other, the diameter of the second fastening part 122b inserted into the through-hole 131 may correspond to the diameter of the through-hole 131 such that the moving member 130 does not move in the diameter direction of the shaft 120, that is, the lateral direction of the shaft 120. For example, a portion of the second fastening part 122b may correspond to the first diameter D₁₃₁ₐ, and the remaining portion of the second fastening part 122b may correspond to the second diameter D_{131b}. That is, the second fastening part 122b may include a portion having a diameter corresponding to the first diameter D₁₃₁ₐ and a portion having a diameter corresponding to the second diameter D_{131b}.

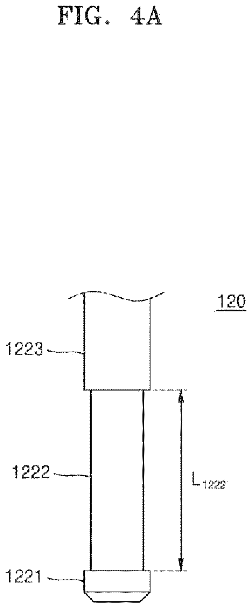

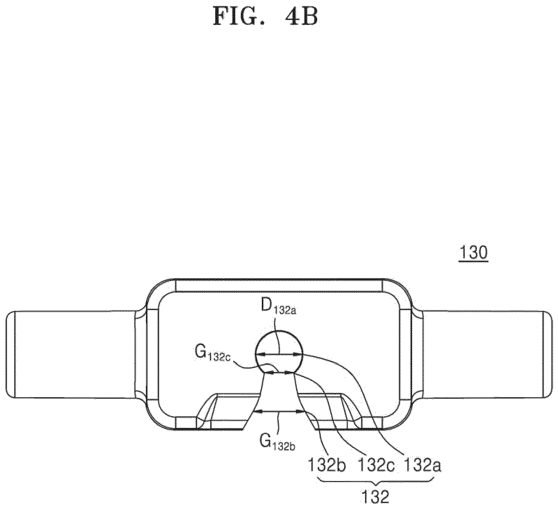

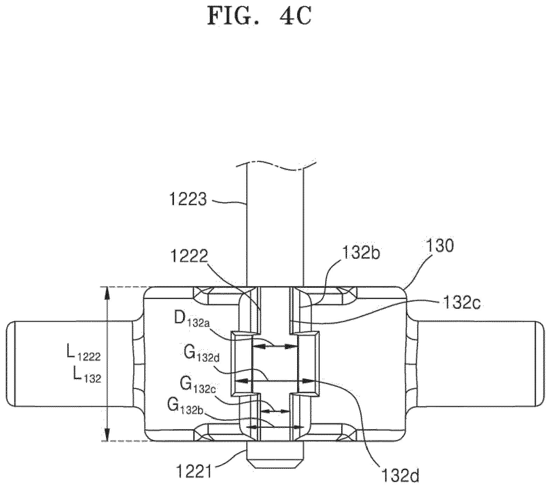

FIG. 4 is a diagram of another embodiment of FIG. 3. FIG. 4A is a diagram illustrating a portion of a second part 122 of a shaft 120, FIG. 4B is a plan view illustrating a moving member 130, and FIG. 4C is a side view illustrating a state in which the shaft 120 and the moving member 130 are coupled to each other.

According to another embodiment of the present disclosure, the shaft 120 and the moving member 130 are coupled to each other by press-fitting, and the shaft 120 may be laterally press-fitted into the moving member 130.

Referring to FIG. 4A, the shaft 120 may include a first fastening part 1221, a second fastening part 1222, and a third fastening part 1223, which are sequentially arranged in the extension direction.

The first fastening part 1221 and the third fastening part 1223 of the shaft 120 may have diameters that are different from a diameter of the second fastening part 1222. For example, the first fastening part 1221 and the third fastening part 1223 may have diameters that are greater than the diameter of the second fastening part 1222.

Referring to FIG. 4B, the moving member 130 may have an insertion groove 132 formed therein. The insertion groove 132 may include an insertion part 132a, a guide part 132b, and an engagement part 132c.

The insertion part 132a may be provided as an elongated hole passing through the moving member 130 from one side to the other side in the central portion of the moving member 130. The insertion part 132a may have a certain diameter D₁₃₂ₐ and a certain length L₁₃₂ₐ.

The guide part 132b connecting the outside of the moving member 130 to the insertion part 132a may be provided such that the shaft 120 outside the moving member 130 is inserted into the insertion part 132a. The shaft 120 may move to the insertion part 132a through the guide part 132b laterally from the outside of the moving member 130.

The guide part 132b may be formed to facilitate the movement of the shaft 120 from the outside of the moving member 130 to the insertion part 132a. For example, the guide part 132b may be expanded and opened in a tapered shape from the insertion part 132a toward the outside of the moving member 130. The opposing surfaces defining the guide part 132b are gradually spaced apart from each other toward the outside of the moving member 130, and a gap G_{132b} of the guide part 132b may increase toward the outside of the moving member 130. The gap G_{132b} of the guide part 132b at the outside may be formed to be greater than the diameter of the insertion part 132a.

The engagement part 132c may be formed at a boundary between the insertion part 132a and the guide part 132b. The engagement part 132c may be provided to prevent the shaft 120 inserted into the insertion part 132a from being separated from the insertion part 132a. For example, the engagement part 132c may be provided with a gap G_{132c} less than the diameter D₁₃₂ₐ of the insertion part 132a.

Referring to FIG. 4C, the shaft 120 may be coupled to the moving member 130 by being press-fitted into the insertion groove 132 formed in the moving member 130 to the side of the shaft 120. For example, the shaft 120 may move laterally through the guide part 132b from the outside of the moving member 130 and may be press-fitted into the insertion groove 132 by a force applied laterally from the engagement part 132c.

In order to facilitate the movement of the shaft 120, the guide part 132b may be formed such that a gap G_{132b} thereof at the outside is greater than the diameter of the second fastening part 1222, and the gap may gradually decrease toward the inside of the moving member 130.

The gap G_{132c} provided by the engagement part 132c formed at the boundary between the guide part 132b and the insertion part 132a is less than the diameter of the second fastening part 1222 such that the engagement part 132c restricts the movement of the shaft 120, and the shaft 120 may be press-fitted into the insertion part 132a through the engagement part 132c by a force laterally applied thereto. The shaft 120 press-fitted into the insertion part 132a may not be separated by the engagement part 132c.

In a state in which the shaft 120 and the moving member 130 are coupled to each other, the second fastening part 1222 may be located in the insertion part 132a. A length L₁₂₂₂ of the second fastening part 1222 may correspond to a length L₁₃₂ₐ of the insertion part 132a, the first fastening part 1221 may be in contact with one side of the moving member 130, and the third fastening part 1223 may be in contact with the other side of the moving member 130. Since the first fastening part 1221 and the third fastening part 1223 have diameters greater than the diameter D132a of the insertion part 132a, such that the movement of the moving member 130 may be restricted to prevent the moving member 130 from moving in the extension direction of the shaft 120.

In a state in which the shaft 120 and the moving member 130 are coupled to each other, the second fastening part 1222 may have a diameter corresponding to the diameter D₁₃₂ₐ of the insertion part 132a such that the moving member 130 may be fixed without shaking to the side of the shaft 120.

The insertion groove 132 may further include an engagement alleviation part 132d. The engagement alleviation part 132d may be formed to facilitate the insertion of the shaft 120 into the insertion part 132a. For example, the engagement alleviation part 132d is formed to be opened outward from the insertion part 132a to the moving member 130, and a gap G_{132d} due to the engagement alleviation part 132d may be provided to be greater than a gap G_{132b} due to the guide part 132b. The gap G_{132d} due to the engagement alleviation part 132d may be formed to be smallest at a portion where the engagement alleviation part 132d and the insertion part 132a are connected to each other, and may be formed to be equal to the diameter D₁₃₂ₐ of at least the insertion part 132a. Accordingly, the engagement part 132c may not be formed at the boundary between the insertion part 132a and the engagement alleviation part 132d.

The engagement alleviation part 132d may reduce a force for press-fitting the shaft 120 into the insertion groove 132 and may reduce stress acting on the moving member 130 when the shaft 120 is press-fitted thereinto.

As such, the coupling method of the shaft 120 and the moving member 130 may facilitate the coupling between the shaft 120 and the moving member 130 and may maintain a tight and stable coupling state between the shaft 120 and the moving member 130. This provides an effect of reducing the time and effort required for manufacturing and coupling and also reducing power loss that may occur at a coupled portion.

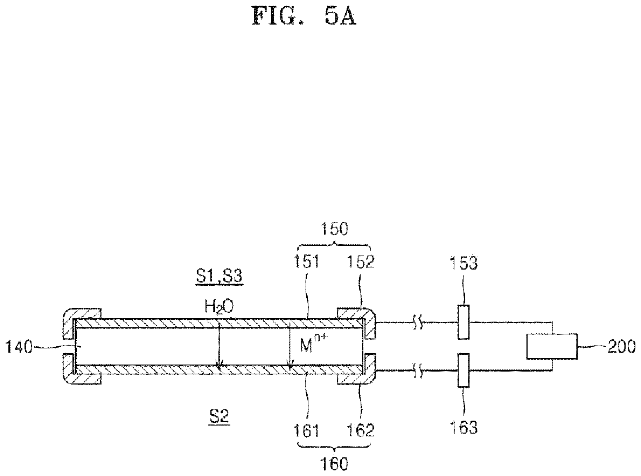

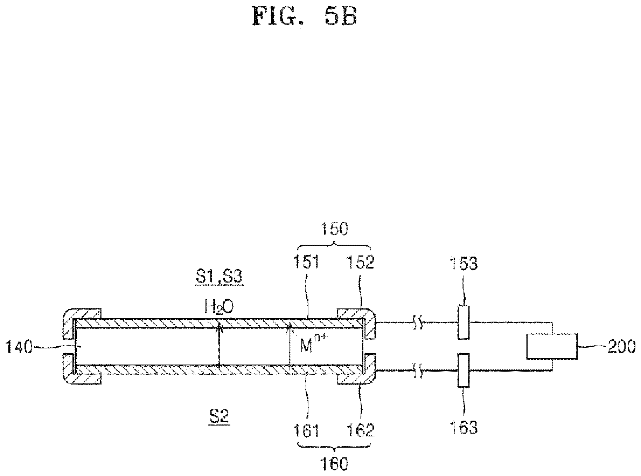

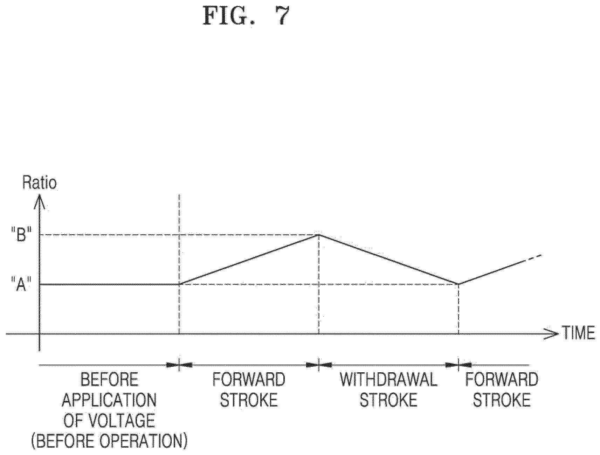

Hereinafter, the behavior of the fluid and the movement of the shaft according thereto are described with reference to FIGS. 5 and 7.

FIGS. 5A and 5B are schematic diagrams illustrating the reaction in the first electrode body 150 and the second electrode body 160 with respect to the membrane.

Referring to FIGS. 5A and 5B, the first electrode body 150 and the second electrode body 160 may be electrically connected to a power supply part 200 through the first terminal 153 and the second terminal 163, respectively. By alternately changing the polarity of the voltage supplied by the power supply part 200, the direction of movement of the liquid such as water may be changed.

A case where silver/silver oxide is used as an electrochemical reactant and the first fluid is a solution including water is described.

As illustrated in FIG. 5A, when the first electrode body 150 is an oxidizing electrode and the second electrode body 160 is a reducing electrode, the reaction of Ag(s)+H2O→Ag2O(s)+2H++2e- occurs in the first electrode body 150, and the reaction of Ag2O(s)+2H++2e-→Ag(s)+H2O occurs in the second electrode body 160.

Cations (Mn+, for example, hydrogen ions) generated according to the oxidation reaction in the first electrode body 150 pass through the membrane 140 and move toward the second electrode body 160 by the voltage difference. At this time, a certain pressure may be generated while water (H2O) is moving together with cations.

Thereafter, when the polarity of the voltage supplied by the power supply part 200 is reversed as illustrated in FIG. 5B, the electrochemical reactant consumed when used as the oxidizing electrode is recovered when used as the reducing electrode. Similarly, the reducing electrode is also recovered. Accordingly, the first electrode body 150 and the second electrode body 160 may continuously react according to the voltage supply of the power supply part 200. Unlike in FIG. 5A, when the polarity of the voltage supplied to the first electrode body 150 and the second electrode body 160 is changed, cations (Mn+, for example, hydrogen ions) and water (H2O) move from the second space S2 back to the first space S1, as illustrated in FIG. 5B.

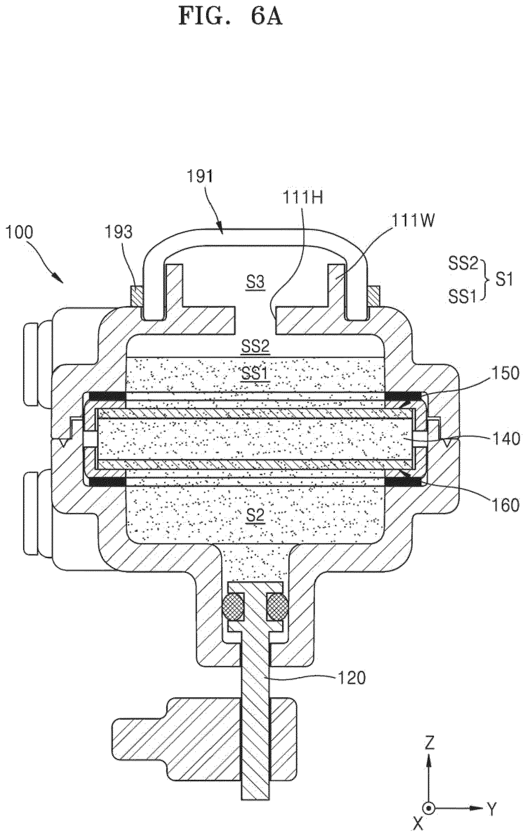

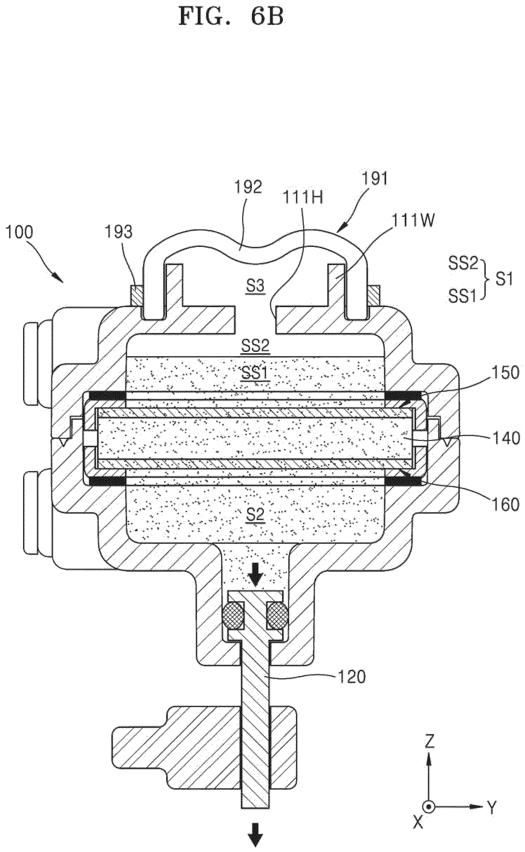

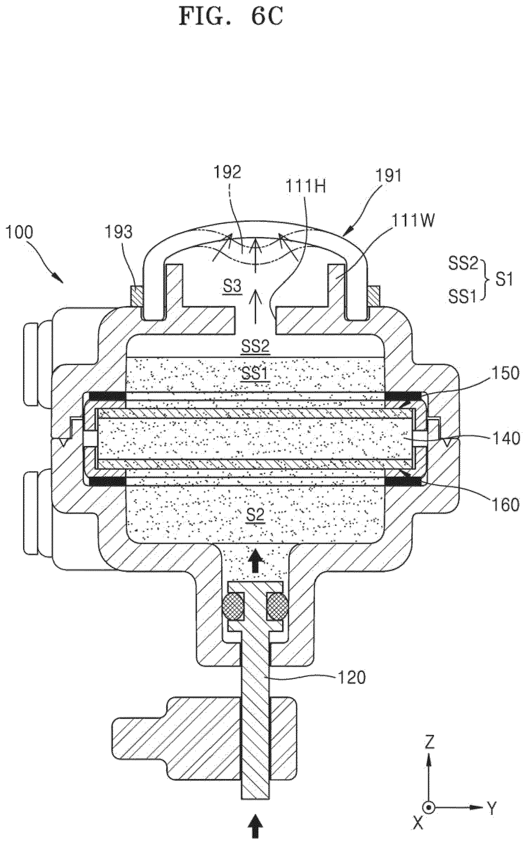

FIGS. 6A to 6C are cross-sectional views describing the reciprocating motion of the shaft. FIG. 6A illustrates a state before the shaft moves, and FIGS. 6B and 6C illustrate a state after the shaft moves. FIG. 6A may be understood as a state before the voltage is applied to the first electrode body 150 and the second electrode body 160 by the power supply part 200 described above with reference to FIG. 5A.

Referring to FIGS. 6A and 6B, the first fluid such as water exists in the inner space of the housing 110, and the volume of the first fluid existing in the inner space is less than the volume of the inner space. The second fluid including a gas such as air may exist in a portion of the inner space where the liquid does not exist, specifically in the first space S1 of the housing 110 and the third space S3 that is the inner space of the deformation part 191.

For example, the first fluid exists in each of the first space S1 and the second space S2. The first fluid and the second fluid coexist in the first space S1, and the volume of the first fluid existing in the first space S1 may be less than the volume of the first space S1.

The first fluid also exists in the second space S2. However, unlike the first space S1, the second fluid does not exist in the second space S2. For convenience of explanation, a portion of the first space S1 where the first fluid, which is a liquid, exists is referred to as a first sub-space SS1, and a portion of the first space S1 where the second fluid, which is a gas, exists is referred to as a second sub-space SS2. The first sub-space SS1 and the second sub-space SS2 may form the first space S1. For example, the remaining portion of the first space S1 other than the first sub-space SS1 may be the second sub-space SS2.

Referring to FIGS. 6A to 6C, the second fluid may exist in the first space S1, specifically in the third space S3 that is the inner space of the deformation part 191 communicating with the second sub-space SS2.

In the state of FIG. 6A, when the power supply part 200 supplies the voltage to the first electrode body 150 and the second electrode body 160 as described with reference to FIG. 5A, the reaction described with reference to FIG. 5A occurs. Cations (e.g., hydrogen ions) move in a first direction (-Z direction in FIG. 6B) from the first space S1 toward the second space S2.

At this time, pressure is generated while the first fluid (e.g., H2O) of the first space S1 pass through the membrane 140 along with cations and move in the first direction (the -Z direction in FIG. 6B), and the shaft 120 moves linearly in the first direction due to the pressure, as illustrated in FIG. 6B.

Referring to FIG. 6A, the deformation part 191 according to an embodiment of the present disclosure may communicate with one side (the upper side in FIG. 6A) of the housing 110 where the first space S1 is formed, and may communicate with the first space S1 and the third space S3, which is the inner space of the deformation part, through the hole 111H formed in one surface (the upper surface in FIG. 6A) of the housing 110 facing the deformation part 191.

Referring to FIG. 2, the main body 111 includes a first sub-body 111A and a second sub-body 111B. The first sub-body 111A and the second sub-body 111B may be coupled to each other with the membrane 140 therebetween.

The hole 111H is formed in the main body 111, specifically in the first sub-body 111A, and the hole 111H is located on the opposite side of the second sub-body 111B that accommodates the shaft 120 with the membrane 140 in the center.

Referring to FIGS. 6A and 6B, an elastic part 192 that is elastically deformable may be formed on one side of the deformation part 191 according to an embodiment of the present disclosure. The elastic part 192 may be formed in the central portion of the deformation part 191 and may be formed to be concave or convex with respect to the outward direction (the upward direction in FIG. 6A) of the deformation part 191.

Referring to FIGS. 6A to 6C, as the shape of the elastic part 192 is deformed, the volume of the third space S3 may increase or decrease.

The shape of the elastic part 192 may be deformed depending on the inner pressure of the inner space of the deformation part 191, specifically the third space S3. Referring to FIG. 6A, negative pressure may be formed in the third space S3.

The elastic part 192 may have an elastic restoring force in a direction in which the elastic part 192 is formed to be convex with respect to the outward direction (the upper direction in FIG. 6A) of the deformation part 191. This provides an effect in which, when the first fluid moves from the second space S2 to the first space S1 together with cations, as illustrated in FIG. 6C, the space in which the second fluid, such as air, is contained is secured as much as the third space S3, and the force required to compress the shaft 120 may be relatively reduced, and the compression is facilitated.

As the first fluid (e.g., H2O) in the first space S1 moves to the second space S2, the volume ratio of the first sub-space SS1 to the volume of the first space S1 decreases, and the proportion occupied by the second sub-space SS2 in the first space S1 increases.

In contrast, referring to FIGS. 6A and 6C, when the power supply part 200 changes the polarity of the voltage and supplies the voltage to the first electrode body 150 and the second electrode body 160 as described with reference to FIG. 5B, cations (e.g., hydrogen ions) and the first fluid (e.g., water) move in a second direction (+Z direction in FIG. 6) from the second space S2 toward the first space S1, and the shaft 120 moves back to an original position thereof as illustrated in FIG. 6A.

Referring to FIG. 6C, while the shaft 120 moves in the second direction (the +Z direction in FIG. 6C), the first fluid moves together with cations in the second direction, and the shape of the deformation part 191, specifically the elastic part 192, may be deformed.

Specifically, as the elastic part 192 is deformed from a concave shape to a convex shape with respect to the outward direction of the deformation part 191, there is an effect in which the volume of the third space S3 increases and the movement and compression of the shaft 120 are facilitated.

In addition, when compression occurs in the first space S1 and the third space S3, the deformation part 191, specifically the elastic part 192, has an elastic restoring force in a direction in which the elastic part 192 is formed to be convex. Accordingly, there is an effect of further facilitating the compression.

In addition, when the reaction in FIGS. 5A and 5B occurs and gas is generated, the gas may be contained in the third space S3. Accordingly, there is an effect of performing a buffer function.

When the power supply part 200 alternately changes the polarity of the voltage applied to the first electrode body 150 and the second electrode body 160, the shaft 120 may reciprocate, such as moving in the first direction, moving in the second direction opposite to the first direction, and then moving again in the first direction.

The reciprocating motion of the shaft 120 may be described as the change according to the volume ratio of the space where the second fluid exists, that is, the second sub-space SS2.

FIG. 7 is a graph showing the ratio of the volume VSS2 of the second sub-space to the volume VS1 of the first space according to the shape deformation of the shaft assembly.

When the ratio (=VSS2/VS1) of the volume of the second sub-space SS2 to the volume of the first space S1 in a state before the power supply part 200 applies voltage to the first electrode body 150 and the second electrode body 160, that is, in a state before the pump 100 operates is "A," the ratio during the forward stroke of the shaft 120, that is, when the shaft 120 moves in the first direction, increases to "B" (A<B, where A is greater than 0 and B is less than 1).

In the stroke where the shaft 120, which had advanced, withdraws in the second direction, the above-described ratio decreases from B to A, but the ratio does not become less than A. When the ratio during the withdrawal stroke becomes less than A, the shaft 120 moves further inside the housing 110, or the inner space of the housing 110, which is a sealed space, is unsealed. Accordingly, problems, such as fluid leakage, may occur.

The pump 100 described with reference to FIGS. 6A to 6C may be a pump in which the respective components (main body, sealing material, etc.) are assembled in a state in which the shaft 120 moves relatively backward. As another embodiment, the pump 100 may be a pump in which the respective components (main body, sealing material, etc.) are assembled in a state in which the shaft 120 moves relatively forward.

When the shaft 120 moves forward or backward, the second fluid (e.g., air) existing in the second sub-space SS2 and the third space S3 may be slightly compressed or slightly expanded. A certain force may be stored in the second fluid depending on the compression or expansion of the second fluid, and this force may act on the shaft 120. Accurate control of the forward and withdrawal strokes of the shaft 120 may affect the amount of medicine injected in the medicine injection device in which the pump 100 is used.

Therefore, the forward and withdrawal strokes of the shaft 120 may be designed, for example, taking into account the aforementioned force.

As described above, referring to FIGS. 1, 2, and 6A to 6C, the deformation part 191 according to an embodiment of the present disclosure communicates with a housing 110 in which the first space S1 is formed. The deformation part 191 may form a third space S3, which is an inner space connected to the first space S1, and the shape thereof may be deformed. The deformation part 191 may be formed of an elastically deformable material, such as silicone.

Specifically, the deformation part 191 may be coupled to the other side (the upper side in FIG. 2) opposite to one side (the lower side in FIG. 2) of the housing 110 in which the shaft 120 reciprocates with respect to the membrane 140.

A hole 111H may be formed in one surface (the upper surface in FIG. 2) of the housing 110 facing the deformation part 191, and the first space S1 and the third space S3 may communicate with each other. This provides an effect of removing the force stored in the second fluid in the first space S1, enabling accurate control of the forward and withdrawal strokes of the shaft 120, compared to forming only the first space S1.

Referring to FIGS. 6A to 6B, the elastic part 192 that is elastically deformable may be formed on one side (the upper side in FIG. 6A) of the deformation part 191 according to an embodiment of the present disclosure. As the elastic part 192 is elastically deformed, the volume of the third space S3 may be deformed.

Referring to FIGS. 6A and 6B, before the reaction of FIG. 5A occurs, negative pressure may be formed in the third space S3, and the shape of the elastic part 192 may be formed to be concave with respect to the outward direction of the deformation part 191.

When the reaction of FIG. 5B occurs as illustrated in FIG. 6C, the pressure increases due to the pressure of the first fluid, etc., and the elastic part 192 has an elastic restoring force in a direction in which the elastic part 192 is formed to be convex with respect to the outward direction of the deformation part 191. Accordingly, there is an effect in which the shape of the deformation part 191 is deformed in a direction in which the volume of the third space S3 increases, compression of the shaft 120 is facilitated by removing the force stored in the second fluid, and accurate control of the shaft 120 is possible.

Referring to FIGS. 1, 2, and 6A to 6C, a fixing member 193 according to an embodiment of the present disclosure is coupled to each of the housing 110 and the deformation part 191, and the position of the deformation part 191 may be fixed on the housing 110.

In addition, there is an effect of blocking the second fluid including air from leaking out of the third space S3 that is the inner space of the deformation part 191.

The fixing member 193 may be in close contact with the outer circumferential surface of the deformation part 191 and may be installed in the housing 110.

Referring to FIGS. 1, 2, and 6A to 6C, a sealing wall 111W that protrudes in the outward direction and is in close contact along the inner circumference of the deformation part 191 may be formed to protrude on one surface (the upper surface in FIG. 2) of the housing 110 facing the deformation part 191.

Due to the sealing wall 111W, the inner circumferential surface of the deformation part 191 is in close contact with the sealing wall 111W, the outer circumferential surface of the deformation part 191 is in close contact with the fixing member 193, and the fluid contained in the third space S3 may be prevented from leaking to the outside.

The pump 100 described with reference to FIGS. 1 to 7 may be a small pump used in a device for injecting medicine such as insulin. However, the use of the pump 100 is not particularly limited as long as the pump 100 linearly moves the shaft 120 by using the structure and mechanism as described above.

The present disclosure has been described with reference to the embodiment illustrated in the drawings, but this is only an example. It will be understood by those of ordinary skill in the art that various modifications and variations may be made thereto. Accordingly, the true technical protection scope of the present disclosure should be defined by the technical spirit of the appended claims.

## Claims

1. An electroosmotic pump comprising:
a housing having a shaft hole;
a shaft passing through the shaft hole, arranged to extend inside and outside the housing, and capable of a reciprocating motion in an extension direction thereof; and
a moving member coupled to the shaft outside the housing,
wherein the shaft is press-fittable into the moving member.

2. The electroosmotic pump of claim 1, wherein the shaft is press-fitted into a through-hole formed in the moving member in the extension direction of the shaft,
a first fastening part, a second fastening part, and a third fastening part are sequentially formed in the extension direction of the shaft at one end portion of the shaft coupled to the moving member,
the first fastening part is contactable with one surface of the moving member,
the third fastening part is contactable with another surface opposite to the one surface of the moving member, and
the second fastening part is located in the through-hole and connects the first fastening part to the third fastening part.

3. The electroosmotic pump of claim 2, wherein the through-hole comprises:
a first through-hole part having a first diameter on one side of the moving member; and
a second through-hole part having a second diameter relatively greater than the first diameter on another side of the moving member, and
wherein a diameter of the first fastening part corresponds to the second diameter, and a diameter of the third fastening part is relatively greater than the second diameter.

4. The electroosmotic pump of claim 3, wherein a portion of the second fastening part corresponds to the first diameter, and a remaining portion of the second fastening part corresponds to the second diameter.

5. The electroosmotic pump of claim 1, wherein the shaft is press-fitted from a side into an insertion groove formed in the moving member,
a first fastening part, a second fastening part, and a third fastening part are sequentially formed in the extension direction of the shaft at one end portion of the shaft coupled to the moving member,
the first fastening part is contactable with one surface of the moving member,
the third fastening part is contactable with another surface opposite to the one surface of the moving member, and
the second fastening part is located in the insertion groove and connects the first fastening part to the third fastening part.

6. The electroosmotic pump of claim 5, wherein the insertion groove comprises:
an insertion part to which the second fastening part is inserted and fixed;
a guide part expanded and opened in an outward direction from the insertion part; and
an engagement part protruding inward between the insertion part and the guide part.

7. The electroosmotic pump of claim 6, wherein a diameter of each of the first fastening part and the third fastening part is relatively greater than a diameter of the insertion part.

8. The electroosmotic pump of claim 6, wherein the insertion groove further comprises an engagement alleviation part that is opened from the insertion part to an outside of the moving member, and a minimum gap formed by the engagement alleviation part is equal to a diameter of the insertion part.
